# EUROPEAN PATENT SPECIFICATION

(11) **EP 4 643 796 B1**
(45) Date of publication and mention of the grant of the patent: **07.10.2026**
(21) Application number: 25173903.3
(22) Date of filing: 02.05.2025
(51) Int. Cl.: A61B 17/34, A61N 1/375

(54) **APPARATUS FOR LOADING A FIBER TETHER IN A BIOSTIMULATOR TRANSPORT SYSTEM**
VORRICHTUNG ZUM LADEN EINES FASERGURTS IN EIN BIOSTIMULATORTRANSPORTSYSTEM
APPAREIL POUR CHARGER UNE ATTACHE DE FIBRE DANS UN SYSTÈME DE TRANSPORT DE BIOSTIMULATEUR

(30) Priority: 03.05.2024 US 202463642474 P; 30.04.2025 US 202519194442
(43) Date of publication of application: 05.11.2025
(73) Proprietor: Pacesetter, Inc., Sylmar, CA 91342 (US)
(72) Inventor: SACHA, Mike, Sylmar, CA 91342 (US); ARNAR, Bernhard, Sylmar, CA 91342 (US)
(74) Representative: Barker Brettell Sweden AB

(56) References cited:
- US-A1- 2016 206 872
- US-A1- 2017 258 346
- US-B2- 10 130 821

## Description

### TECHNICAL FIELD

The disclosed embodiments relate generally to biostimulators. More specifically, but not exclusively, the disclosed embodiments relate to transport systems used to implant biostimulators such as leadless pacemakers in a patient.

### BACKGROUND

Cardiac pacing by an artificial pacemaker, known more generally as a "biostimulator," provides electrical stimulation of the heart when the heart's own natural pacemaker and/or conduction system fails to provide synchronized atrial and ventricular contractions at healthy rates and intervals. Such antibradycardial pacing provides relief from symptoms, and even life support, for hundreds of thousands of patients. Cardiac pacing can also provide electrical overdrive stimulation to suppress or convert tachyarrhythmias, again supplying relief from symptoms and preventing or stopping arrhythmias that could lead to sudden cardiac death.

Currently available or conventional pacemakers usually perform cardiac pacing using an electrical pulse generator implanted subcutaneously or sub-muscularly in or near a patient's pectoral region. The pulse generator's operating parameters are usually interrogated and modified in one of three ways: by a programming device outside the body, by a loosely-coupled transformer with one inductance inside the body and another outside, or by electromagnetic radiation with one antenna inside the body and another outside. The pulse generator usually connects to the proximal end of one or more implanted leads, the distal ends of which contain one or more electrodes for positioning adjacent to the inside or outside wall of a cardiac chamber. The leads have an electrical conductor for connecting the pulse generator to electrodes in the heart. Such electrode leads typically have lengths of 50-70 centimeters. Pacing leads can engage and/or be fixed to an intracardial implant site by an engaging mechanism such as an electrode anchor. For example, the electrode anchor can screw into the myocardium.

Leadless cardiac pacemakers locate all electronic circuitry at the pacing site, thereby eliminating electrical leads and avoiding shortcomings associated with conventional cardiac pacing systems. Leadless cardiac pacemakers can be anchored at a pacing site-for instance, in a right ventricle for single-chamber pacing and in both a right ventricle and a right atrium for dual-chamber pacing-by an anchor. A delivery system can be used to deliver the leadless cardiac pacemakers to the target anatomy and a recovery system, which in some instances can be the same as the delivery system, is used to retrieve a leadless pacemaker. US2016206872A1 pertains to interventional medical systems, and more particularly to delivery tools and related assemblies that are configured to facilitate percutaneous transvenous deployment of relatively compact implantable medical devices.

### SUMMARY

In one aspect, a loading tool for a biostimulator transport system, the loading tool comprises a plunger having a free end and a tether end. A tether is coupled to the tether end of the plunger and coupled to a biostimulator. A storage container has an outlet, and the plunger is stored in the storage container and can exit the storage container through the outlet.

In an embodiment, the storage container includes a spool having a cylindrical hub and a pair of parallel spaced-apart flanges, each flange coupled to an end of the cylindrical hub, and a housing surrounding an outer perimeter of the spool, the outlet being formed in the housing.

In an embodiment, one flange of the pair of flanges includes a receptacle adapted to receive the biostimulator.

In an embodiment, the plunger and the tether are wound around the hub.

In an embodiment, the tether is stored in the storage container with the plunger.

In an embodiment, the tether end of the plunger includes a tether coupler.

In an embodiment, the tether coupler is an eye formed at the tether end of the plunger.

In an embodiment, the loading tool comprises a base and the storage container is formed on the base and adapted to store the plunger.

In an embodiment, the loading tool further comprises a catheter receptacle coupled to the base to receive a distal end of a delivery catheter.

In an embodiment, the receptacle is positioned so that the plunger can be fed into the distal end of the delivery catheter as the plunger exits the storage container.

In an embodiment, the storage container includes a housing formed on the base and having an outlet formed therein, and a spool having a cylindrical hub and a pair of spaced-apart parallel flanges. Each flange is coupled to an end of the cylindrical hub, and the spool is rotatable relative to the housing and is positioned in the housing so that the housing surrounds an outer perimeter of the spool.

In an embodiment, the plunger is semi-rigid and is coiled around the hub.

In an embodiment, the loading tool further includes a tether coupled to the tether end of the plunger and coupled to a biostimulator.

In an embodiment, the tether is coiled around the hub together with the plunger.

In an embodiment, the loading tool further includes a feed mechanism coupled to the base to remove the plunger from the spool.

In an embodiment, the feed mechanism is to feed the plunger into the distal end of the delivery catheter.

In a further aspect, a biostimulator transport system includes a delivery catheter having a distal end and a loading tool according to the invention.

In an embodiment, the loading tool includes a base, a storage container formed on the base and adapted to store a plunger, and a receptacle coupled to the base to receive the distal end of the delivery catheter. The receptacle is positioned so that the plunger can be fed into the distal end of the delivery catheter as it exits the storage container.

In an embodiment, the storage container includes a housing formed on the base, the housing having an outlet formed therein, and a spool having a cylindrical hub and a pair of spaced-apart parallel flanges. Each flange is coupled to an end of the cylindrical hub, and the spool is rotatable relative to the housing and is positioned in the housing so that the housing surrounds an outer perimeter of the spool.

In an embodiment, the biostimulator transport system further includes a plunger having a free end and a tether end, wherein the plunger is coiled around the hub.

In an embodiment, the biostimulator transport system further includes a tether coupled to the tether end of the plunger and coupled to a biostimulator.

In an embodiment, the tether is coiled around the hub together with the plunger.

In an embodiment the biostimulator transport system further includes a feed mechanism coupled to the base to remove the plunger from the spool and feed it into the distal end of the delivery catheter.

### BRIEF DESCRIPTION OF THE DRAWINGS

The invention is defined in claim 1. Non-limiting and non-exhaustive embodiments of the present disclosure are described with reference to the following figures, wherein like reference numerals refer to like parts throughout the various views unless otherwise specified. Figures are not to scale unless otherwise indicated.
Fig. 1 is a diagrammatic cross section of a human heart illustrating an embodiment of implantation of a biostimulator in a target anatomy.
Fig. 2 is a perspective view of an embodiment of a biostimulator system including a biostimulator and a biostimulator transport system.
Figs. 3A-3E are views of embodiments of a plunger for loading a fiber tether into a biostimulator transport system.
Figs. 4A-4C are top views of a biostimulator transport system that together show an embodiment of a process for loading a fiber tether into the catheter.
Figs. 5A-5C are top views of a biostimulator transport system that together show another embodiment of a process for loading a fiber tether into the catheter.
Figs. 6A-6B are perspective views of embodiments of loading tools for loading a fiber tether into a biostimulator transport system.
Figs. 7A-7B are a perspective view and a plan view, respectively, of another embodiment of a loading tool for loading a fiber tether into a biostimulator transport system.
Figs. 8A-8B are, respectively, a plan view of embodiment of a loading tool for loading a fiber tether into a biostimulator transport system and a side view of an embodiment of a feed mechanism for use with the loading tool.

### DETAILED DESCRIPTION

Embodiments are described of an apparatus, system, and method for loading a tether into a biostimulator transport system used for delivery of biostimulators such as leadless pacemakers. Specific details are described to provide an understanding of the embodiments, but one skilled in the relevant art will recognize that the disclosure can be practiced without one or more of the described details or with other methods, components, materials, etc. In some instances, well-known structures, materials, or operations are not shown or described in detail but are nonetheless encompassed within the scope of the disclosure.

Reference throughout this specification to "one embodiment" or "an embodiment" means that a described feature, structure, or characteristic can be included in at least one described embodiment, so that appearances of "in one embodiment" or "in an embodiment" do not necessarily all refer to the same embodiment. Furthermore, the described features, structures, or characteristics can be combined in any suitable manner in one or more embodiments.

As used in this application, directional terms such as "left," "right," "front," "rear," "upper," lower," "top," "bottom," "side," "lateral," "longitudinal," etc., refer to the orientations of embodiments as they are presented in the drawings, but any directional term should not be interpreted to imply or require a particular orientation of the described embodiments when in actual use. The use of relative terms throughout the description can denote a relative position or direction. For example, "distal" can indicate a first direction along a longitudinal axis of a biostimulator transport system and "proximal" can indicate a second direction opposite to the first direction. Such terms are provided to establish relative frames of reference, but are not intended to limit the use or orientation of a biostimulator transport system to a specific configuration described in the various embodiments below.

Implanting a biostimulator such as a leadless pacemaker (LP) in a patient's heart is accomplished using a biostimulator transport system, e.g., a delivery system that is usually, but not necessarily, a delivery catheter. Implantation of the LP is typically a multi-step process including at least one or more of these steps:
- **Preparation.** One end of the LP (also known as the LP's "button") is attached to a flexible tether and the tether is pulled into the interior of the catheter until the button is engaged by the catheter's end cap. The end cap is positioned at the catheter's distal end and is essentially a socket that can engage and release the LP and that allows the catheter to exert forces and torques on the LP during implantation. Preparation can also include loading the flexible tether into a distal end of the catheter and routing it to a proximal end of the catheter.
- **Insertion and Implantation.** The LP, now attached to the distal end of the catheter, is inserted in the patient's body and transported by the catheter through the body to the specific heart tissue where it will be implanted. Once there, the LP's attachment mechanism is used to attach the LP to the heart tissue. In one embodiment, fixation to the heart tissue is achieved by mechanical means such as a screw-in helical screw or tines (small hooks extending from the LP).
- **Tether Mode.** After the LP is attached to the heart tissue, the LP is released from the catheter's end cap but remains attached to the flexible tether. The purpose of this "tether mode" is to evaluate the LP's attachment to the heart while retaining the ability to retrieve the LP by pulling it back into engagement with the end cap if the LP attachment proves unsatisfactory. Because the tether is flexible, tether mode allows the LP to move along with the heart as it beats, simulating the loads and movements the LP will face when implantation is complete.
- **LP Release and Catheter Removal.** If the LP's attachment to the heart tissue is satisfactory, then the flexible tether is detached from the LP's button and the tether is partially or fully pulled into the catheter's interior until it is no longer attached to the LP, leaving the LP in the heart. The catheter, now without the LP on its end, is transported back out of the patient.
- **Repeat.** In a case where multiple LPs will be implanted in the patient's heart, some or all of the steps above can be repeated. Otherwise, the procedure is substantially complete.

The embodiments disclosed and discussed below are directed to improving this process. Existing tethering systems use thin metal wires, usually made of nitinol, to tether the LP to the delivery catheter. These wire tethers suffer from metal fatigue, require expensive precision machining, and are subject to unwanted offset due to arc length differences. But one advantage of a wire tether-based system is its reloadability: the rigidity of wire tethers makes them easier to reload into a catheter-i.e., having used one wire tether to deliver an LP, it is easier to insert another wire tether into the catheter so that the catheter can be reused to deliver another LP. This can be important for dual-chamber cases where LPs are placed in both the right ventricle and right atrium. Fiber-based tethers provide increased fatigue resistance, are significantly cheaper, and do not require precise machining. But fiber tethers, because they are rigid and cannot support a compression load without buckling, are difficult to load into a delivery catheter. The described embodiments provide reloadability of a fiber-based tether system using a novel loading tool.

There are several embodiments of the loading tool, all based on the same underlying principle which uses a plunger or mandrel to provide structural support to string the fiber through the delivery catheter. The fiber/plunger is spooled inside a novel loading tool to reduce space, and variations of the loading tool can also aid in the insertion of the fiber by using a mechanism to automatically advance the plunger. This system offers several advantages, apart from the obvious one of being able to reload a fiber-based tether in the delivery catheter. It also solves the issues of operating room table space and keeping surgical instruments within the sterile field, because the lengthy fibers/plungers are spooled up and stored within a small package. Furthermore, some embodiments discussed below can facilitate the tether loading process for the smallest footprint from a space perspective, while also removing the hassle of manually inserting the plunger through the catheter. Having a robust tether system that is reloadable also reduces cost in a dual-chamber case because only one catheter is needed for both implants.

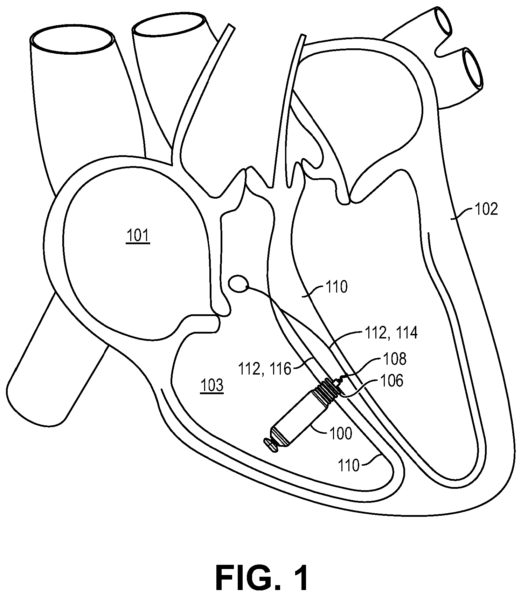

Fig. 1 illustrates an embodiment of implantation of a biostimulator in a target anatomy of a human heart. A leadless biostimulator system, e.g., a cardiac pacing system, includes one or more biostimulators 100. Biostimulator 100 can be implanted in a patient heart 102, and can be leadless (e.g., it can be a leadless pacemaker (LP)). Each biostimulator 100 can be placed in a cardiac chamber, such as a right atrium 101 and/or right ventricle 103 of heart 102, or attached to an inside or outside of the cardiac chamber. For example, biostimulator 100 can be attached to a septum 110 of heart 102. More particularly, biostimulator 100 can be delivered to the septum 110, and one or more elements, such as a fixation element 106 and/or a pacing element 108, can pierce a septal wall 110 to engage and anchor the biostimulator 100 to the target anatomy, e.g., a bundle branch 112 in the septal wall 110. In a particular embodiment, biostimulator 100 can use two or more electrodes located on or within a housing of the biostimulator 100 for pacing the cardiac chamber upon receiving a triggering signal from at least one other device within the body. In an embodiment, one or more of the fixation element 106 or the pacing element 108 is an active electrode.

When biostimulator 100 is delivered to and attached to the target tissue, e.g., an atrial or ventricular wall, or a septum 110 of the heart 102, pacing element 108 and/or fixation element 106 can be positioned for pacing. For example, in the case of deep septal pacing at respective bundle branches 112 in septum 110, an active electrode of pacing element 108 can be positioned at a first target anatomy in the septal wall 110, e.g., a left bundle branch 114. Similarly, the fixation element 106 can be positioned at a second target anatomy in the septal wall, e.g., a right bundle branch 116. Optionally, one of the elements may be at a bundle branch and the other element may not be at a bundle branch.

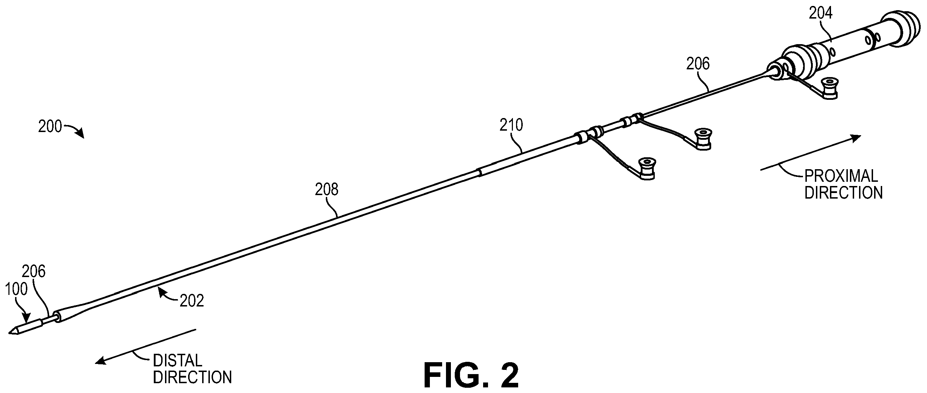

Fig. 2 illustrates an embodiment of a biostimulator system 200. Biostimulator system 200 can include a biostimulator 100, e.g., a leadless pacemaker or other leadless biostimulator. System 200 can also include delivery or retrieval systems, which may be catheter-based systems used to carry biostimulator 100 intravenously to or from a patient anatomy. For example, a biostimulator transport system 202 can be used to deliver biostimulator 100 to, or retrieve the biostimulator from, a patient. Biostimulator 100 can be attached, connected to, or otherwise mounted on biostimulator transport system 202. The biostimulator can be mounted on a distal end of a the biostimulator transport system 202, which can include a delivery catheter used to advance biostimulator 100 intravenously into or out of heart 102. Biostimulator transport system 202 may alternatively include a retrieval catheter used to retrieve biostimulator 100 from the heart 102.

Biostimulator transport system 202 can include a handle 204 to control movement and operations of the transport system from outside a patient. One or more elongated members extend distally from handle 204. For example, an elongated catheter body 206 can extend distally from handle 204 to a distal end of the biostimulator transport system. In an embodiment, biostimulator 100 is mounted on a distal end of elongated catheter body 206.

Biostimulator transport system 202 can include a protective sleeve 208 to cover biostimulator 100 during delivery and implantation. Protective sleeve 208 can extend over, and be longitudinally movable relative to, elongated catheter body 206. The biostimulator transport system can also include an introducer sheath 210 that can extend over, and be longitudinally movable relative to, protective sleeve 208. Introducer sheath 210 can cover a distal end of protective sleeve 208, elongated catheter body 206, and biostimulator 100 as those components are passed through an access device into the patient anatomy.

Several components of biostimulator transport system 202 are described above, but the biostimulator transport system can be configured to include additional or alternate components. More particularly, biostimulator transport system 202 can be configured to deliver biostimulator 100 to, or retrieve it from, the target anatomy. Delivery and/or retrieval of biostimulator 100 can include retaining the biostimulator 100 on catheter 102 during transport to the target anatomy and rotation of the biostimulator during its implantation at the target anatomy. Accordingly, biostimulator transport system 202 can incorporate features to retain and rotate biostimulator 100.

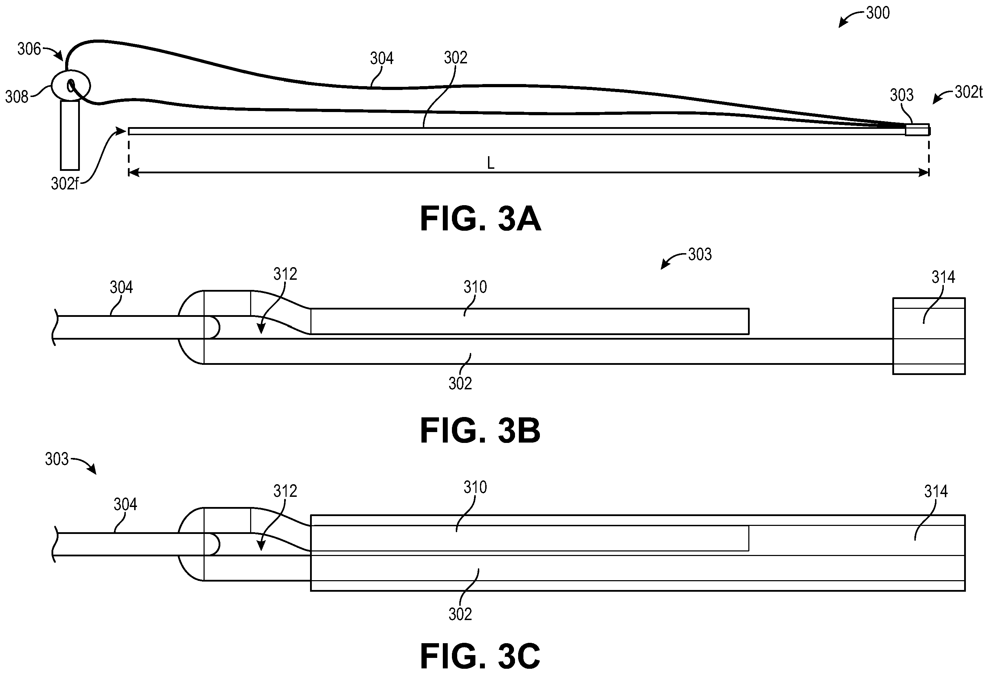

Figs. 3A-3D together illustrate an embodiment of a plunger arrangement 300 for loading a fiber tether into, e.g., a delivery catheter of a biostimulator transport system. In plunger arrangement 300, a plunger 302 has a free end 302f, a tether end 302t, and a length L that is substantially the distance between the free end and the tether end. A tether coupler 303 is formed at the tether end 302t so that a tether 304 can be coupled to the tether end.

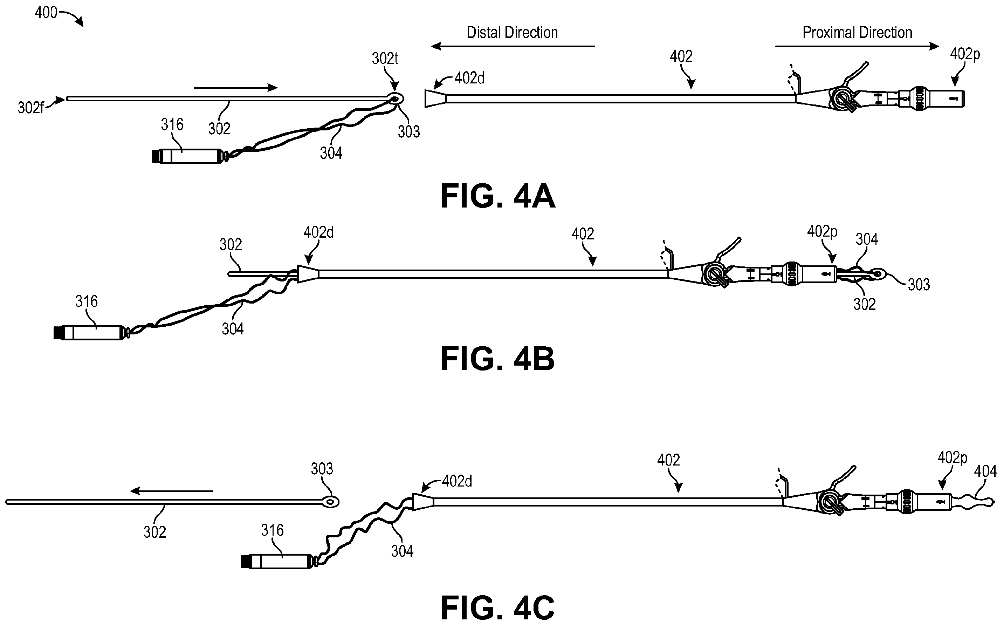

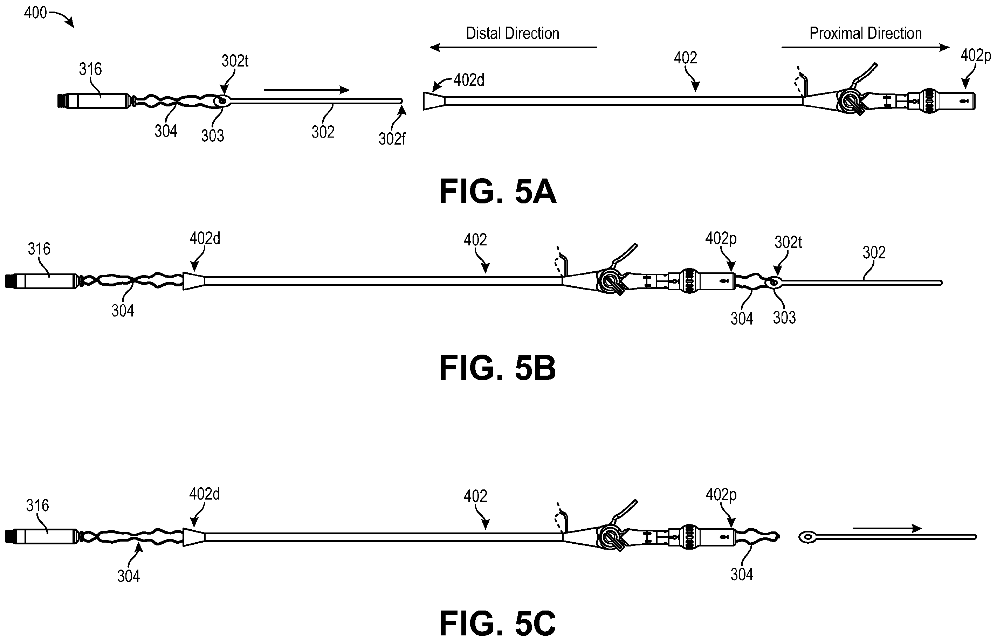

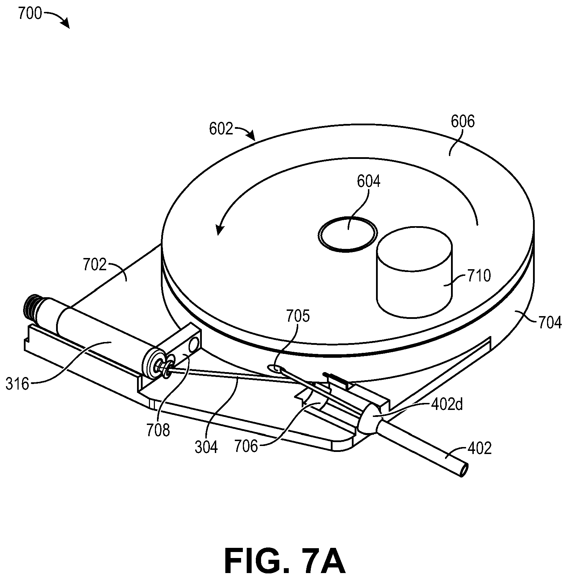

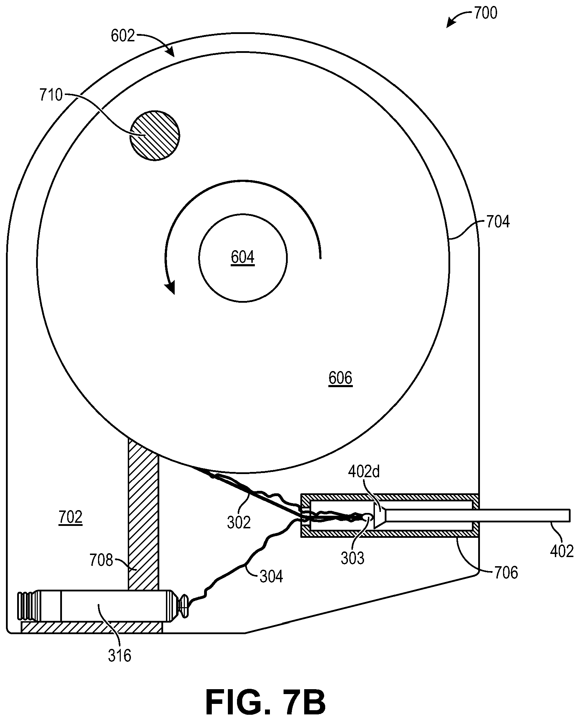

In one embodiment plunger 302 is semi-rigid, meaning that it is stiff enough to sustain at least a small compression load without buckling, but flexible enough that it can be elastically deformed or otherwise bent to be stored in a storage container (see, e.g., Figs. 7-9). In one embodiment plunger 302 can be made of nitinol, but in other embodiments of can be made of a different material. Length L will generally be at least as long as the length of a catheter in which the plunger will be inserted (see, e.g., Figs. 4A-4C and 5A-5C).

Tether 304 is a fiber tether that, in the illustrated embodiment, is formed into a loop. The loop is slidably coupled to tether coupler 303 and is also slidably coupled to an end cap 308 of a biostimulator, for instance through a hole in the end cap. The tether is slidably coupled to end cap 308, meaning that the tether is constrained by the end cap but can slide though it, so that the biostimulator can be easily released from the tether once the biostimulator is satisfactorily secured in a patient. In one embodiment tether 304 is also slidably coupled to tether coupler 303, but in other embodiments it need not be slidably coupled to tether coupler 303. In some embodiments, tether 304 can be made of a fibrous, polymeric, non-metallic material. For instance, in one embodiment the tether can be made from commonly available surgical suture materials such as Ultra High Molecular Weight Polyethylene (UHMWPE) and Polyester (PE). Other embodiments can use other materials, such as Kevlar^{®}, Vectran^{®}, etc. These materials offer benefits including high tensile strength, high flexibility and suppleness, virtually infinite fatigue life, and low cost.

Figs. 3B-3C illustrate an embodiment of a tether coupler 303. In the illustrated embodiment, tether coupler 303 is formed by bending a tip of plunger 302 back in the direction of the plunger, so that a small section 310 abuts, and is substantially parallel to, plunger 302. When bent this way, an eye 312 is formed at the end of the plunger. Tether 304 can then be coupled to tether coupler 303, and hence to plunger 302, by threading the tether through eye 312. A collar or cincture tube 314 can slide back and forth along the plunger between an open position, shown in Fig. 3B, and a closed position shown in Fig. 3C. In the closed position, the collar helps retain tether 304 in eye 312 by preventing the tether from sliding out between plunger 302 and parallel section 310. In the open position, the collar allows the tether to be removed by sliding it out between plunger 302 and parallel section 310. In other embodiments tether coupler 303 can be formed differently than shown; for instance, in one embodiment tether coupler 303 can be a hole formed directly in an end of plunger 302, analogously to how an eye is formed at the end of a sewing needle.

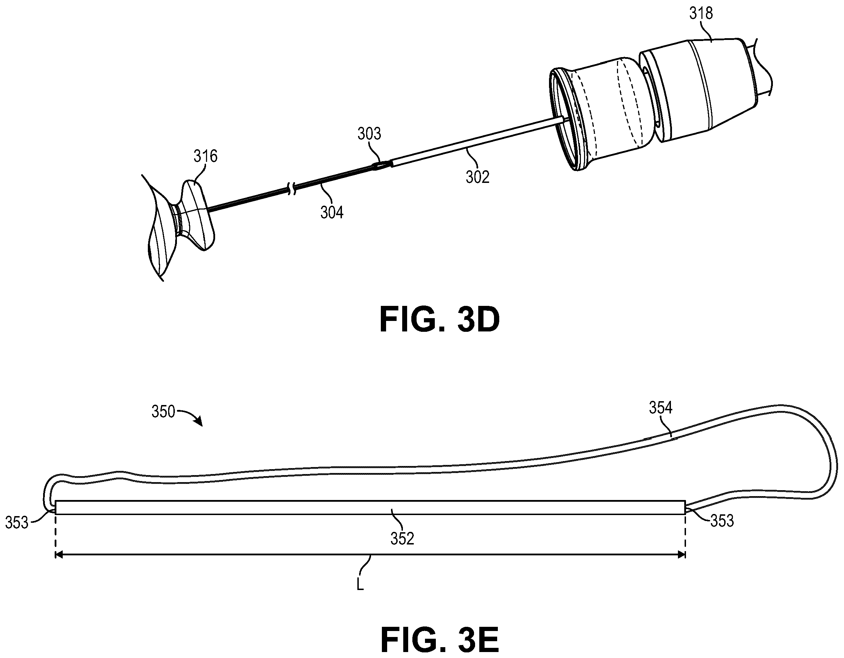

Fig. 3D illustrates a plunger 302 with a tether 304 coupled to tether coupler 303 and to a biostimulator 316. In this arrangement, tether 304 is loaded into a catheter 318 by pushing and/or pulling the tether into and through the catheter, as discussed in more detail below in connection with Figs. 4A-4C and 5A-5C. In some embodiments, the surgeon or other person assisting with the operation can manually couple the tether to the biostimulator, the tether coupler, or both. But in other embodiments the fiber tether can come pre-coupled to the biostimulator, the tether coupler, or both.

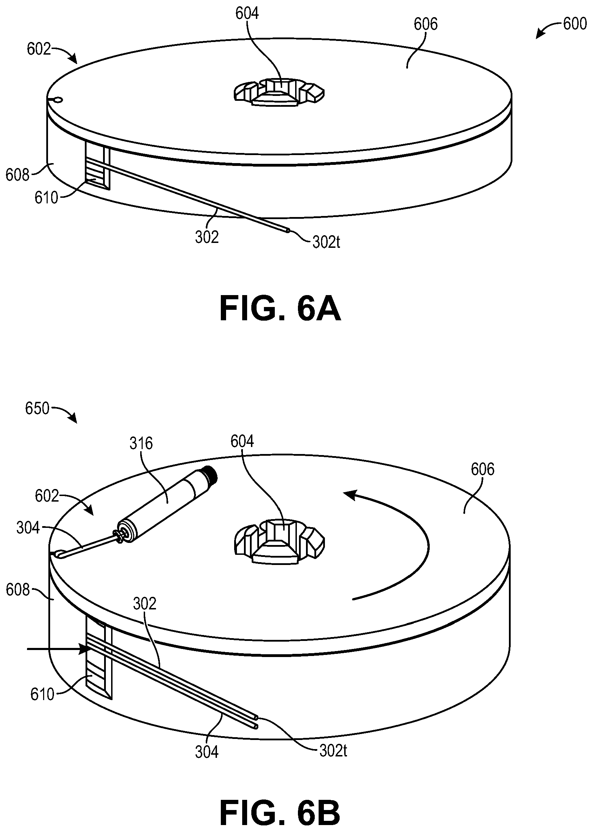

Fig. 3E illustrates another embodiment of a plunger arrangement 350. Plunger arrangement 350 includes a plunger 352 which, like plunger 302, is semi-rigid, meaning that it is stiff enough to sustain at least a small compression load without buckling, but flexible enough that it can be elastically deformed or otherwise bent to be stored in a storage container (see, e.g., Figs. 6A et seq.). In one embodiment plunger 352 can be made of nitinol, but in other embodiments of can be made of a different material. Length L will generally be at least as long as the length of a catheter in which the plunger will be inserted. But unlike plunger 302, plunger 352 has a tether coupler 353 at each end, so that a tether 354 can be attached to both ends of the plunger. In one embodiment, tether couplers 353 are similar to tether couplers 303, but in other embodiments the tether couplers can be different. Also, in one embodiment both ends of plunger 352 can have the same tether coupler, but in other embodiment both ends need not have the same tether coupler. Plunger arrangement 350 operates substantially as described below for plunger arrangement 300.

Figs. 4A-4C together illustrate an embodiment of a process 400 for using a plunger to load a fiber tether into, e.g., a delivery catheter of a biostimulator transport system that is part of a biostimulator delivery system. A fiber tether can carry little or no compression load without buckling, so the illustrated embodiment uses a plunger such as plunger 302 described above, to load a fiber tether 304 into a catheter 402.

Fig. 4A illustrates an initial part of the process, which starts with plunger 302 having tether 304 coupled to tether coupler 303 and biostimulator 316. Plunger 302 is inserted, tether end 302t first, into a distal end 402d of catheter 402 and is pushed in a proximal direction. As plunger 302 moves through the catheter with tether 304 coupled to tether coupler 303, the tether is pulled into the catheter while biostimulator 316 remains coupled to the tether outside distal end 402d.

Fig. 4B illustrates a next part of the process. Starting as shown in Fig. 4A, after tether end 302f of the plunger is inserted into distal end 402d, the plunger is pushed in a proximal direction until its tether end, and hence tether coupler 303 and tether 304, emerge from proximal end 402p of the catheter. At this stage, tether 304 extends from outside distal end 402d, through the interior of catheter 402, to outside the proximal end 402p.

Fig. 4C illustrates a final part of the process. Starting as shown in Fig. 4B, after tether coupler 303 emerges from proximal end 402p, tether 304 is removed from the tether coupler. The tether can optionally be fixed at or near proximal end 402p using some form of locking mechanism. Plunger 302 is then removed from the catheter. In the illustrated embodiment, plunger 302 is removed by pulling it in a distal direction until it fully emerges from distal end 402d, but in another embodiment plunger 302 could instead be pulled in a proximal direction until it fully emerges from proximal end 402p, as shown in Fig. 5C. Either way, tether 304, with biostimulator 316 still attached at one end, now extends fully through the length of catheter 402 and tether loading is substantially complete.

Figs. 5A-5C together illustrate an embodiment of another process 500 for using a plunger to load a fiber tether into, e.g., a delivery catheter of a biostimulator transport system that is part of a biostimulator delivery system.

Fig. 5A illustrates an initial part of the process, which starts with plunger 302 having tether 304 coupled to tether coupler 303 and biostimulator 316. Plunger 302 is inserted, free end 302f first, into a distal end 402d of catheter 402 and is pushed in a proximal direction until the free end emerges from proximal end 402p. As plunger 302 moves through the catheter with tether 304 coupled to tether coupler 303, the tether is pulled into the catheter while biostimulator 316 remains coupled to the tether outside distal end 402d.

Fig. 5B illustrates a next part of the process. Starting as shown in Fig. 5A, when free end 302f of plunger 302 emerges from proximal end 402p, the plunger is pulled in a proximal direction until tether end 302t, tether coupler 303, and tether 304 emerge from proximal end 402p. As a result, tether 304 now extends from outside distal end 402d, through the interior of catheter 402, to outside proximal end 402p.

Fig. 5C illustrates a final part of the process. Starting as shown in Fig. 5B, after tether coupler 303 emerges from proximal end 402p, tether 304 is removed from the tether coupler. The tether can optionally be fixed at or near the proximal end of the catheter using some form of locking mechanism. Plunger 302, now fully out of the catheter, is then put away or otherwise disposed of. Tether 304, with biostimulator 316 still attached at one end, now extends fully through the length of catheter 402 and tether loading is substantially complete.

Figs. 6A-6B illustrate embodiments of loading tools 600 and 650 for loading a fiber tether into a catheter. Until plunger 302 and/or tether 304 are needed for an implant procedure, loading tools 600 and 650 mostly function as a storage receptacle for the plunger and/or the tether. In the embodiment of Fig. 6A, only plunger 302 is stored in the tool (i.e., coiled on spool 602), but in the embodiment of Fig. 6B both plunger 302 and tether 304 are stored in the tool.

Fig. 6A illustrates an embodiment of a loading tool 600. Loading tool 600 includes a spool 602 with a hub 604 and a pair of parallel spaced-apart flanges 606 coupled to the hub (only one flange 606 is visible in this drawing). In one embodiment both flanges 606 can be of the same size, but in other embodiments they need not be. Spool 602 is mounted in a housing 608, so that the housing surrounds and encloses the perimeter of the spool. When mounted in housing 608, spool 602 can rotate about the center of hub 604 relative to the housing. Housing 608 includes an outlet 610 through which a plunger 302 can emerge from the housing.

Plunger 302 is within the tool, coiled around hub 604. In the illustrated embodiment, the plunger can be coiled around hub 604 free-end-first, so that free end 302f is closest to the hub and tether end 302t is farthest from the hub; in this configuration, tether end 302t is first to emerge from outlet 610 when the plunger is unloaded from the tool. In another embodiment, the plunger can be coiled around hub 604 tether-end-first, so that tether end 302t is closest to the hub and free end 302f is farthest from the hub; in this configuration, free end 302f is first to emerge from outlet 610 when the plunger is removed.

Loading tool 600 can operate in two primary modes, depending mostly on how the plunger is coiled around hub 604. Both modes relieve the surgeon of dealing with a long, floppy plunger; minimize the needed table space; and help keep all the surgical instruments within the sterile field.

The first mode of operation, used where the plunger is coiled in tool 600 free-end-first, is mostly as shown in Figs. 4A-4C. A small section of the plunger's tether end 302t can be manually pulled out of the tool by the surgeon. If tether 304 (not shown in this drawing, but see Fig. 6B) is already attached to tether coupler 303 and wound onto the spool with the plunger, then tether is pulled out of the spool along with the plunger. Otherwise, a tether 304 can be coupled to the plunger's tether coupler 303 and to a biostimulator. Tether end 302t, with tether 304 attached to its tether coupler, is then slowly fed through the distal end of a catheter and into its interior. The plunger and/or tether can be unspooled from tool 600 as needed when the plunger is pushed further into the catheter until tether end 302f and/or tether 304 emerge from the proximal end of the catheter.

The second mode of operation, used where the plunger is coiled in tool 600 tether-end-first, is mostly as shown in Figs. 5A-5C. A small section of the free end 302f is manually pulled out of the tool and inserted into the distal end of a catheter. Free end 302f is fed through the distal end 402d of catheter 402 and into its interior, unspooling the plunger from tool 600 as needed, until the free end emerges from the proximal end of the catheter. If tether 304 is not already coupled to tether coupler 303, the part of the plunger remaining in the tool is unspooled until tether coupler 303 is exposed, at which point a tether 304 (not shown in this drawing, but see Fig. 6B) can be coupled to the tether coupler and to a biostimulator. But if tether 304 is already attached to tether coupler 303 and coiled on the spool with the plunger, in this second mode the tether will usually be coiled onto the spool before the plunger, so that tether only starts to emerge from the tool once the tether coupler emerges from the tool. Either way, the tether is loaded into the catheter by pulling the plunger from the proximal end of the catheter until the tether coupler and the tether emerge from the proximal end of the catheter.

Fig. 6B illustrates another embodiment of a loading tool 650. Loading tool 650 is in most respects similar to loading tool 600. The primary differences between loading tools 600 and 650 are that in loading tool 650 both the plunger and the tether are coiled onto spool 602. Loading tool 650 also includes a receptacle to hold a biostimulator 316. In the illustrated embodiment the receptacle is formed in flange 606, but in other embodiments the receptacle can be formed elsewhere on the tool. Also in the illustrated embodiment biostimulator 316, when positioned in the receptacle, is already attached to an end of tether 304. Loading tool 650 can operated in the same two modes discussed above for loading tool 600.

Figs. 7A-7B together illustrate an embodiment of a loading tool 700 for loading a fiber tether into, e.g., a delivery catheter of a biostimulator transport system. Fig. 7A is a perspective view, Fig. 7B a plan view. Loading tool 700 includes a base 702 on which is formed a housing 704, and housing 704 includes an outlet 705 through which plunger 302 and/or tether 304 can emerge. A receptacle or nest 706 is formed on the base to receive and removably hold a distal end 402d of a delivery catheter 402, and another receptacle 708 is formed on the base to removably hold a biostimulator 316.

A spool 602 is mounted in housing 704, so that the housing surrounds and encloses the perimeter of the spool. Spool 602 includes a hub 604 and a pair of parallel spaced-apart flanges 606 coupled to the hub (only one flange 606 is visible in these drawings). In one embodiment both flanges 606 can be of the same size, but in other embodiments they need not be. When mounted in housing 704, spool 602 can rotate about the center of hub 604 relative to housing 704 and base 702. A knob 710 can optionally be formed on flange 606 so that the spool can be rotated by grasping the knob and moving it in a circular motion around hub 604, as indicated by the arrow in the figure.

In operation of loading tool 700, the tether end of plunger 302, with an end of tether 304 coupled to the tether coupler, is positioned at an end of receptacle 706. The other end of tether 304 is coupled to biostimulator 316. In one embodiment these initial steps can be done manually, but in other embodiments the biostimulator can come pre-packaged in receptacle 706 and the plunger and tether can come pre-positioned at the end of receptacle 706. After these two steps are done, loading tool 700 operates similarly to loading tools 600 and 650, as discussed above. The primary difference in operation is that loading tool 700 requires less manual work because the loading tool includes provisions for holding the distal end of catheter 402 and the biostimulator 316, and because it includes a mechanism, such as knob 710, for rotating spool 602. Rotating spool 602 this way unloads the plunger and the tether from the spool and pushes them into catheter 402.

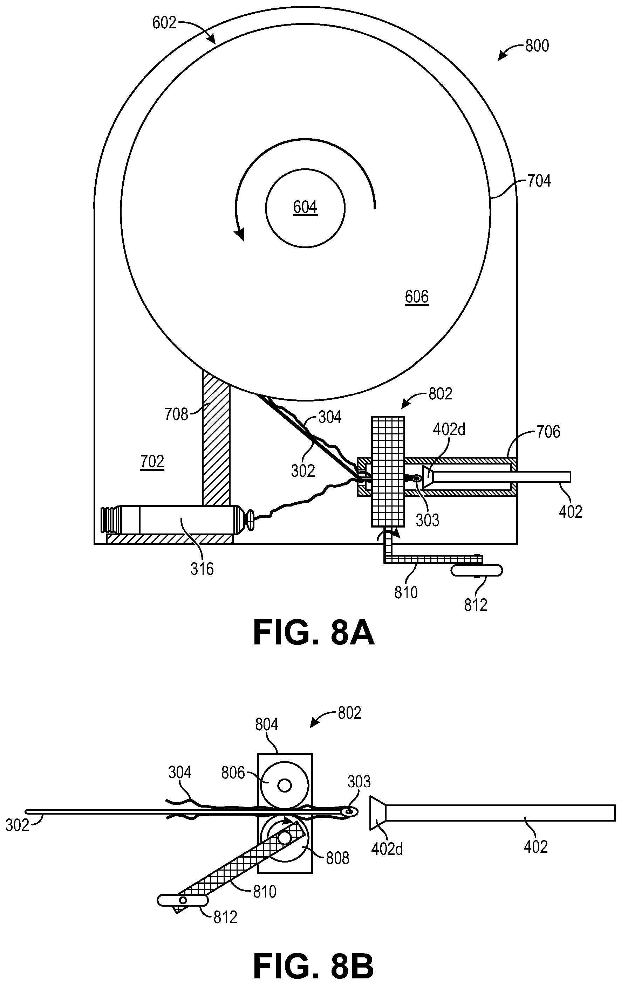

Figs. 8A-8B illustrates an embodiment of a loading tool 800 for loading a fiber tether into a catheter. Fig. 8A illustrates a loading tool 800 that is in most respects similar to loading tool 700 described above and operates similarly. The primary difference between loading tools 700 and 800 is that loading tool 800 includes a feed mechanism 802 to pull plunger 302 and/or tether 304 out of spool 602 and feed them into catheter 402. In the illustrated embodiment, feed mechanism 802 is positioned on base 702 spanning over receptacle 706, but in other embodiments the feed mechanism can be positioned elsewhere on the tool.

Fig. 8B illustrates an embodiment of a feed mechanism 802. The feed mechanism include a housing 804 with a pair of friction wheels 806 and 808 inside. Friction wheels 806 and 808 both rotate about their respective centers and are positioned so that there is a small gap between their perimeters to engage plunger 302 and tether 304. In the illustrated embodiment, friction wheel 806 can rotate freely while friction wheel 808 is coupled to a crank arm 810 and crank handle 812. The crank arm and crank handle can be used to turn friction wheel 808, thus drawing plunger 302 and tether 304 into the gap between friction wheels and pushing the plunger and tether into catheter 402 through distal end 402d, as described above. In other embodiments, friction wheel 808 can rotate freely and crank arm 810 and crank handle 812 can instead be coupled to friction wheel 806. Still other embodiments can use an entirely different kind of feed mechanism.

## Claims

1. A loading tool (600, 650, 700, 800) for a biostimulator transport system (202), the loading tool (600, 650) comprising:
a plunger (302) having a free end (302f) and a tether end (302t);
a tether (304) coupled to the tether end (302t) of the plunger (302) and coupled to a biostimulator (316); and
a storage container (602, 608, 704) having an outlet (610), wherein the plunger (302) is stored in the storage container (602, 608, 704) and can exit the storage container (602, 608, 704) through the outlet (610, 706).

2. The loading tool of claim 1, wherein the storage container (602, 608, 704) includes:
a spool (602) having a cylindrical hub (604) and a pair of parallel spaced-apart flanges (606), each flange (606) coupled to an end of the cylindrical hub (604); and
a housing (608, 704) surrounding an outer perimeter of the spool (602), the outlet (610, 706) being formed in the housing (608, 706).

3. The loading tool of claim 2, wherein one flange of the pair of flanges (606) includes a receptacle adapted to receive the biostimulator (316).

4. The loading tool of claim 2 or 3, wherein the plunger (302) and the tether (304) are wound around the cylindrical hub (604).

5. The loading tool of any one of claims 1 to 4, wherein the tether (304) is stored in the storage container (602, 608) with the plunger (302).

6. The loading tool of any one of claims 1 to 5, wherein the tether end (302t) of the plunger (302) includes a tether coupler (303).

7. The loading tool of claim 6, wherein the tether coupler (303) is an eye (312) formed at the tether end (302t) of the plunger (302).

8. The loading tool of any one of claims 1 to 7, further comprising:
a base (702); and
the storage container (602, 704) formed on the base (702) and adapted to store the plunger (302).

9. The loading tool of claim 8, further comprising:
a catheter receptacle (706) coupled to the base (702) to receive a distal end (402d) of a delivery catheter (402).

10. The loading tool of claim 9, wherein the catheter receptacle (706) is positioned so that the plunger (302) can be fed into the distal end (402d) of the delivery catheter (402) as the plunger (302) exits the storage container (602, 704).

11. The loading tool of any one of claims 8 to 10, wherein the storage container (602, 704) includes:
a housing (704) formed on the base (702), the housing (704) having an outlet (706) formed therein; and
a spool (602) having a cylindrical hub (604) and a pair of spaced-apart parallel flanges (606), each flange (606) coupled to an end of the cylindrical hub (604), the spool (602) being rotatable relative to the housing (704) and positioned in the housing (704) so that the housing (704) surrounds an outer perimeter of the spool (602).

12. The loading tool of any one of claims 8 to 11, further comprising a feed mechanism (802) coupled to the base (702) to remove the plunger (302) from the spool (602).

13. The loading tool of claim 12, wherein the feed mechanism (802) is to feed the plunger (302) into the distal end (402d) of the delivery catheter (402).

14. The loading tool of any one of claims 1 to 13, wherein the plunger (302) is semi-rigid and is coiled around the cylindrical hub (604).

15. A biostimulator transport system (202) comprising:
the loading tool (700, 800) of claim 9 or 10; and
the delivery catheter (402) having the distal end (402d).

## Patentansprüche

1. Ladewerkzeug (600, 650, 700, 800) für ein Biostimulatortransportsystem (202), wobei das Ladewerkzeug (600, 650) Folgendes umfasst:
einen Kolben (302), der ein freies Ende (302f) und ein Gurtende (302t) aufweist;
einen Gurt (304), der mit dem Gurtende (302t) des Kolbens (302) gekoppelt und mit einem Biostimulator (316) gekoppelt ist; und
einen Lagerbehälter (602, 608, 704), der einen Auslass (610) aufweist, wobei der Kolben (302) in dem Lagerbehälter (602, 608, 704) verstaut ist und über den Auslass (610, 706) aus dem Lagerbehälter (602, 608, 704) austreten kann.

2. Ladewerkzeug nach Anspruch 1, wobei der Lagerbehälter (602, 608, 704) Folgendes beinhaltet:
eine Spule (602), die eine zylindrische Nabe (604) und ein Paar paralleler beabstandeter Flansche (606) aufweist, wobei jeder Flansch (606) mit einem Ende der zylindrischen Nabe (604) gekoppelt ist; und
ein Gehäuse (608, 704), das einen Außenumfang der Spule (602) umgibt, wobei der Auslass (610, 706) in dem Gehäuse (608, 706) ausgebildet ist.

3. Ladewerkzeug nach Anspruch 2, wobei ein Flansch des Paars von Flanschen (606) ein Behältnis beinhaltet, das dazu angepasst ist, den Biostimulator (316) aufzunehmen.

4. Ladewerkzeug nach Anspruch 2 oder 3, wobei der Kolben (302) und der Gurt (304) um die zylindrische Nabe (604) gewickelt sind.

5. Ladewerkzeug nach einem der Ansprüche 1 bis 4, wobei der Gurt (304) mit dem Kolben (302) in dem Lagerbehälter (602, 608) verstaut ist.

6. Ladewerkzeug nach einem der Ansprüche 1 bis 5, wobei das Gurtende (302t) des Kolbens (302) einen Gurtkoppler (303) beinhaltet.

7. Ladewerkzeug nach Anspruch 6, wobei der Gurtkoppler (303) ein Auge (312) ist, das an dem Gurtende (302t) des Kolbens (302) ausgebildet ist.

8. Ladewerkzeug nach einem der Ansprüche 1 bis 7, ferner umfassend:
eine Basis (702); und
den Lagerbehälter (602, 704), der auf der Basis (702) ausgebildet und dazu angepasst ist, den Kolben (302) zu verstauen.

9. Ladewerkzeug nach Anspruch 8, ferner umfassend:
ein Katheterbehältnis (706), das mit der Basis (702) gekoppelt ist, um ein distales Ende (402d) eines Abgabekatheter (402) aufzunehmen.

10. Ladewerkzeug nach Anspruch 9, wobei das Katheterbehältnis (706) so positioniert ist, dass der Kolben (302) in das distale Ende (402d) des Abgabekatheters (402) eingeführt werden kann, wenn der Kolben (302) aus dem Lagerbehälter (602, 704) austritt.

11. Ladewerkzeug nach einem der Ansprüche 8 bis 10, wobei der Lagerbehälter (602, 704) Folgendes beinhaltet:
ein Gehäuse (704), das auf der Basis (702) ausgebildet ist, wobei das Gehäuse (704) einen darin ausgebildeten Auslass (706) aufweist; und
eine Spule (602), die eine zylindrische Nabe (604) und ein Paar von beabstandeten parallelen Flanschen (606) aufweist, wobei jeder Flansch (606) mit einem Ende der zylindrischen Nabe (604) gekoppelt ist, wobei die Spule (602) relativ zu dem Gehäuse (704) drehbar und so in dem Gehäuse (704) positioniert ist, dass das Gehäuse (704) einen Außenumfang der Spule (602) umgibt.

12. Ladewerkzeug nach einem der Ansprüche 8 bis 11, ferner umfassend einen Zuführmechanismus (802), der mit der Basis (702) gekoppelt ist, um den Kolben (302) aus der Spule (602) zu entfernen.

13. Ladewerkzeug nach Anspruch 12, wobei der Zuführmechanismus (802) dazu dient, den Kolben (302) in das distale Ende (402d) des Abgabekatheters (402) einzuführen.

14. Ladewerkzeug nach einem der Ansprüche 1 bis 13, wobei der Kolben (302) halbstarr ist und um die zylindrische Nabe (604) gewickelt ist.

15. Biostimulatortransportsystem (202), umfassend:
das Ladewerkzeug (700, 800) nach Anspruch 9 oder 10; und
wobei der Abgabekatheter (402) das distale Ende (402d) aufweist.

## Revendications

1. Outil de chargement (600, 650, 700, 800) pour un système de transport de biostimulateur (202), l'outil de chargement (600, 650) comprenant :
un piston (302) présentant une extrémité libre (302f) et une extrémité d'attache (302t) ;
une attache (304) reliée à l'extrémité d'attache (302t) du piston (302) et reliée à un biostimulateur (316) ; et
un récipient de stockage (602, 608, 704) présentant une sortie (610), dans lequel le piston (302) est stocké dans le récipient de stockage (602, 608, 704) et peut sortir du récipient de stockage (602, 608, 704) à travers la sortie (610, 706).

2. Outil de chargement selon la revendication 1, dans lequel le récipient de stockage (602, 608, 704) comprend :
une bobine (602) présentant un moyeu cylindrique (604) et une paire de brides (606) parallèles espacées l'une de l'autre, chaque bride (606) étant reliée à une extrémité du moyeu cylindrique (604) ; et
un boîtier (608, 704) entourant un périmètre externe de la bobine (602), la sortie (610, 706) étant ménagée dans le boîtier (608, 706).

3. Outil de chargement selon la revendication 2, dans lequel une bride de la paire de brides (606) comprend un réceptacle apte à recevoir le biostimulateur (316).

4. Outil de chargement selon la revendication 2 ou 3, dans lequel le piston (302) et l'attache (304) sont enroulés autour du moyeu cylindrique (604).

5. Outil de chargement selon l'une quelconque des revendications 1 à 4, dans lequel l'attache (304) est stockée dans le récipient de stockage (602, 608) avec le piston (302).

6. Outil de chargement selon l'une quelconque des revendications 1 à 5, dans lequel l'extrémité d'attache (302t) du piston (302) comprend un coupleur d'attache (303).

7. Outil de chargement selon la revendication 6, dans lequel le coupleur d'attache (303) est un œillet (312) ménagé au niveau de l'extrémité d'attache (302t) du piston (302).

8. Outil de chargement selon l'une quelconque des revendications 1 à 7, comprenant en outre :
une base (702) ; et
le récipient de stockage (602, 704) formé sur la base (702) et apte à stocker le piston (302).

9. Outil de chargement selon la revendication 8, comprenant en outre :
un réceptacle de cathéter (706) relié à la base (702) pour recevoir une extrémité distale (402d) d'un cathéter d'administration (402).

10. Outil de chargement selon la revendication 9, dans lequel le réceptacle de cathéter (706) est positionné de sorte que le piston (302) peut être introduit dans l'extrémité distale (402d) du cathéter d'administration (402) à mesure que le piston (302) sort du récipient de stockage (602, 704).

11. Outil de chargement selon l'une quelconque des revendications 8 à 10, dans lequel le récipient de stockage (602, 704) comprend :
un boîtier (704) formé sur la base (702), le boîtier (704) présentant une sortie (706) ménagée dans celui-ci ; et
une bobine (602) présentant un moyeu cylindrique (604) et une paire de brides (606) parallèles espacées l'une de l'autre, chaque bride (606) étant reliée à une extrémité du moyeu cylindrique (604), la bobine (602) étant apte à tourner par rapport au boîtier (704) et étant positionnée dans le boîtier (704) de sorte que le boîtier (704) entoure un périmètre externe de la bobine (602).

12. Outil de chargement selon l'une quelconque des revendications 8 à 11, comprenant en outre un mécanisme d'alimentation (802) relié à la base (702) pour retirer le piston (302) de la bobine (602).

13. Outil de chargement selon la revendication 12, dans lequel le mécanisme d'alimentation (802) est destiné à introduire le piston (302) dans l'extrémité distale (402d) du cathéter d'administration (402).

14. Outil de chargement selon l'une quelconque des revendications 1 à 13, dans lequel le piston (302) est semirigide et est enroulé autour du moyeu cylindrique (604).

15. Système de transport de biostimulateur (202) comprenant :
l'outil de chargement (700, 800) selon la revendication 9 ou 10 ; et
le cathéter d'administration (402) présentant l'extrémité distale (402d).
